# EUROPEAN PATENT SPECIFICATION

(11) **EP 0 910 344 B1**
(45) Date of publication and mention of the grant of the patent: **16.04.2003**
(21) Application number: 97929241.4
(22) Date of filing: 19.06.1997
(51) Int. Cl.: A61K 9/16, A61K 9/20

(54) **GRANULATE FOR THE PREPARATION OF FAST-DISINTEGRATING AND FAST-DISSOLVING COMPOSITIONS CONTAINING A HIGH AMOUNT OF DRUG**
GRANULATE FÜR DIE VORBEREITUNG VON SCHNELLZERFALLENDE UND SCHNELLLÖSENDEZUBEREITUNGEN MIT HOHEM ANTEIL AN WIRKSTOFF
GRANULES DESTINES A LA PREPARATION DE COMPOSITIONS A HAUT POUVOIR DE DESINTEGRATION ET DE DISSOLUTION, A TENEUR ELEVEE EN MEDICAMENT

(30) Priority: 03.07.1996 EP 96201829; 20.12.1996 US 770421
(43) Date of publication of application: 28.04.1999
(73) Proprietor: YAMANOUCHI EUROPE B.V., 2350 AC Leiderdorp (NL)
(72) Inventor: DIJKGRAAF, Bernardus, Leonardus, Johannes, P.O. Box 108 NL-2350 AC Leiderdorp (NL); MÜHLENBRUCH, Aart, P.O. Box 108 NL-2350 AC Leiderdorp (NL)
(86) International application number: PCT/EP97/03250
(87) International publication number: WO 98/001114

(56) References cited:
- EP-A- 0 281 200
- EP-A- 0 330 284
- WO-A-92/19227
- DE-A- 4 310 963
- FR-A- 2 318 621
- GB-A- 1 560 475
- US-A- 4 911 921
- O'CONNOR R.E. ET AL: "Spheronization II : drug release from drug diluent mixtures" DRUG DEV. IND. PHARM. SCI., vol. 11, no. 9-10, 1985, PHILADELPHIA, pages 1837-1857, XP000613242
- ELBERS J.A.C. ET AL: "Effect of amount and composition of granulation liquid on mixing, extrusion and spheronization" DRUG DEV. IND. PHARM., vol. 18, no. 5, 1992, pages 501-517, XP000613400
- BARATO G. ET AL: "Evaluation of apparatus for granule production. A practical example in the pharmaceutical industry" LABO-PHARMA-PROBL. TECH., vol. 342, 1984, pages 401-401, XP000613532
- 'Martindale', 1982, THE PHARMACEUTICAL PRESS, LONDON

## Description

The present invention relates to a granulate, containing an active ingredient, having a solubility in water of 1:>10, in admixture with a water dispersible cellulose, and fast-disintegrating and fast-dissolving compositions, containing the said granulate.

### BACKGROUND OF THE INVENTION

It is well-known that for an effective treatment of diseases high doses of drugs and especially of antimicrobial compounds may have to be administered. In addition thereto there is a need to reduce the dosing frequency from 4 or 3 times daily to twice or once daily in order to increase patient compliance. The daily dose of a drug may thus be divided over 1 or 2 instead of 3 or 4 doses, which means that the amount of drug per dosage-form has to be increased. Some drugs, depending on their potency and pharmacokinetic properties can be suitably incorporated in so-called modified-release preparations. Other drugs, such as e.g. amoxicillin, appear to provide the best bioavailability when incorporated in compositions having an immediate release of the active ingredient only (Scand. J. Gastroenterol. (1996) 31(1), pages 49-53). Since it is not convenient for a patient to take at the same time two or more small preparations containing the same medicament instead of one large preparation, especially not for the elderly who normally have to take multiple medications, there always has been a need for such easily swallowable dosage-forms, containing a maximum amount of drug and a minimum amount of excipients and still having the desired fast-disintegrating and fast-dissolving properties. Numerous attempts to realise such dosage-forms have been undertaken since the seventies, but as it appeared the compositions and manufacturing methods developed were applicable to certain selected drugs only.

I.M. Jalal et al. in J. Pharm Sci (1972) 61:9, pages 1466-1467, disclose tablets, containing 80% of a drug which is magnesium hydroxide, sulphadiazine or acetaminophen, and 20% of a binder which is Avicel® RC581 (microcrystalline cellulose and sodium carboxymethyl cellulose). Tablets were prepared by mixing the drug and the said binder, granulating the blend with water, drying the granules and compressing these into tablets. Disintegration times varied from 0.8 minutes (magnesium hydroxide) to 3.5 minutes (sulphadiazine). In the present inventors' experience it has become clear that the results greatly depend on the properties of the drug: e.g. when they applied the above method to prepare tablets containing 80 wt% of amoxicillin trihydrate, a disintegration time of 12½ minutes was observed, tablets containing ibuprofen disintegrated after more than 5 minutes and tablets containing sulphameltioxazole required 35 minutes to disintegrate in water. The results, as obtained by Jalal for sulphadiazine and acetaminophen, could be rather well reproduced by the present inventors.

O'Connor et al. in Drug Devel. Ind. Pharm (1985) 11(9&10) pages 1837-1857 evaluated in-vitro drug release profiles for uncoated pellets, consisting of different drugs and diluents (microcrystalline cellulose and two types of water dispersible cellulose) in varying ratio's and prepared by wet granulation and subsequent extrusion and spheronisation. However, fast dissolution rates could not be observed at all.

G. Barato et al. in Labo-Pharma (1984) 32, no 342 pages 401-403 describe granulates, consisting of a high amount of a drug having a water solubility of 1:>10, carboxymethyl cellulose and polyvinylpyrrolidone and being prepared by conventional wet granulation. Tablets made out of the granulate were reported to disintegrate after 13 minutes only.

In DE-4310963 the preparation of alleged fast-release tablets of propafenon-HCl was disclosed. According to figure 7 the percentage release of active ingredient after 30 minutes was 10% only.

US 4911921 discloses tablets, containing a high dose of ibuprofen. The tablets could be prepared by conventional wet granulation of the drug, a binder and a wet granulation binding agent. Although a fast-release of the active ingredient could be reached, tablets did not disintegrate rapidly.

A.A. Chalmers et al. in J. Pharm. Pharmac. (1976) 28, pages 234-238, disclose tablet formulations containing a high amount (>90%). of oxytetracycline and optionally intragranular microcrystalline cellulose (7.2%) and/or alginic acid (2.7%). The tablets were prepared either by slugging or by the conventional wet granulation technique, using water or a 2.5 wt% solution of polyvinylpyrrolidone in water. Very fast disintegration times could be reached by tablets prepared by the slugging method only, provided also that alginic acid was incorporated in the granulate. When these techniques were applied to e.g. amoxicillin, also an antibiotic compound, no fast-disintegrating tablets could be obtained.

British patent 1560475 discloses tablets containing more than 90% by weight of an orally active cephalosporin and consequently less than 10 wt% of excipients (binder, disintegrant and lubricant). The tablets can be prepared by admixing the cephalosporin and excipients and then compressing the mixture, but preferably the tablets are prepared by dissolving or dispersing the binder in water or a suitable organic solvent and using this liquid to granulate the cephalosporin. The dried granules are then mixed with the disintegrant and the lubricant and compressed into tablets. However, the disintegration time of such tablets as measured in distilled water at 37°C ranged from 2 minutes to 13 minutes.

DE-2259646 discloses tablets containing about 90% of a cephalosporin compound and about 10% of excipients (crystalline cellulose, ultraamylopectin and a lubricant). The tablets are prepared by mixing the above ingredients, making a dry granulate and compressing the granules so obtained into tablets. The tablets were said to disintegrate in artificial gastric juice at 37°C within 3 minutes.

EP-0200252 discloses tablets containing 80-98% of a mixture, consisting of trimethoprim or a salt thereof and a sulfonamide or a salt thereof in a ratio between 1 : 20 and 20 : 1, and an ion-exchange compound of the artificial resin type, such as Amberlite® IRP 88. The tablets are prepared by a wet granulation technique using a solution containing a wet granulation binding agent in ethanol or a mixture of ethanol and water. Disintegration times were reported to be less than 1 minute in general, but the teachings of this application appeared to be not applicable to other drugs or combinations of drugs.

A formulation technique which is generally applicable to different drugs, having a solubility in water of less than 10% is disclosed in EP-0330284-B. The drug is blended with 20-100 wt% of a cellulose product, which is microcrystalline cellulose, microfine cellulose or a mixture thereof, and granulated with an aqueous solution, containing up to 0.5 wt% of a wet granulation binding agent, all percentages based on the weight of the active ingredient. After drying and sieving the granules they may be blended with suitable excipients, such as disintegrants, lubricants and flavours, to obtain fast-disintegrating tablets. A specific example thereof is disclosed in EP-0281200-B, which describes fast-disintegrating tablets, containing an amphoteric beta-lactam antibiotic, 24-70 wt% of a first disintegrant, which is microcrystalline cellulose and/or microfine cellulose, and a second disintegrant. Disintegration times were reported to be within one minute in general. The bioavailability of e.g. amoxicillin trihydrate showed to be as good as that of a commercially available suspension and was independent of the way of administering the tablet (as a suspension after having allowed the tablet to disintegrate in water or as a swallowable tablet). However, in the present inventors' experience this formulation technique appeared to be not applicable for the preparation of fast-disintegrating and fast-dissolving tablets, containing a higher amount of active ingredient and a consequently considerably lower amount of the cellulose product.

WO 92/19227 discloses tablet formulations, having a high drug content. The tablets were prepared by dry compaction techniques, including the preparation of the granulate. Although apparently fast-disintegrating tablets could be obtained, it was not clear which method was used for the assessment and which requirements were to be met. No results of dissolution studies were provided.

The problem to be solved by the present invention was to provide a composition or manufacturing method, which would be generally applicable to all kinds of active ingredients having a solubility in water of less than 10%, in order to obtain fast-disintegrating and fast-disssolving compositions, containing a high amount of the active ingredient, while using conventional production equipment.

### SUMMARY OF THE INVENTION

The object of the present invention is to provide a granulate, containing an active ingredient, having a solubility in water of 1:>10, in admixture with ≤ 15 wt%, most preferably 2-5 wt%, of a water dispersible cellulose, the percentage based on the weight of the said active ingredient.

Another object of the present invention is to provide fast-disintegrating and fast-dissolving compositions, containing the said granulate.

### DETAILED DESCRIPTION OF THE INVENTION

It has now been found that by making a blend of an active ingredient, having a solubility in water of 1:>10, in admixture with ≤ 15 wt% of a water dispersible cellulose, the percentage based on the weight of the active ingredient, granulating the blend with water or an aqueous solution and subsequent drying of the wet granulate a granulate is obtained which can be advantageously used for the preparation of fast-disintegrating and fast-dissolving compositions.

The water dispersible cellulose to be used is also known as "microcrystalline cellulose and carboxymethyl cellulose sodium", e.g. in the U.S. Pharmacopoeia/National Formulary. Microcrystalline cellulose is a partially purified depolymerised form of α-cellulose and is obtained by treating pulps derived from fibrous plant material with dilute mineral acid solutions. The acid preferentially attacks the less ordered or amorphous regions of the cellulose polymer chain, thereby exposing and freeing the crystalline sites which form cellulose crystallite aggregates. The reaction mixture is washed to remove the degraded byproducts, the resulting wet-cake freed of water and spray-dried to form dry porous particles having a broad size distribution. Microcrystalline cellulose is a white, odorless, tasteless, relatively free-flowing powder, insoluble in water, organic solvents, dilute alkalies and dilute acids. It finds wide use as a pharmaceutical excipient, viz. as a binder/ diluent in the manufacture of tablets by direct compression and wet granulation, as a tablet disintegrant, as a tablet glidant/ anti-adherent and as a capsule diluent. Microcrystalline cellulose is commercially available in different particle size grades with different properties, e.g. Avicel® PH 101 and 102. Carboxymethyl cellulose sodium is prepared by the action of sodium monochloro acetate on alkalised cellulose. It is a white to faintly yellow, colorless, hygroscopic powder or granular material and is commercially available in a number of different grades with respect to degree of substitution, viscosity, particle size etc.. It is well-known as a suspending and/or viscosity increasing agent and a wet granulation binding agent. The cross-linked product finds use as a tablet disintegrant. The water dispersible celluloses are colloidal forms of microcrystalline cellulose, prepared by chemical depolymerisation of highly purified wood pulp, the original crystalline areas of the fibres being combined with sodium carboxymethyl cellulose and spray-dried. These also find wide use as a pharmaceutical and cosmetic excipient, viz as an oil-in-water emulsifier, an emulsion or foam stabilising agent, as a suspending agent in pharmaceutical suspensions (ready-made as well as reconstitutable suspension) and as a thickening agent. The water dispersible celluloses are also regarded as excellent excipients in compositions, which are prepared by wet granulation and subsequent extrusion and spheronisation, because the sodium carboxymethyl cellulose component improves the binding properties and the plasticity of the mass to be extruded. The water dispersable celluloses thus can be advantageously used in the preparation of compositions having a sustained-release of the active ingredient. Four types of the said celluloses have been marketed under the trade names Avicel® RC-501 (containing 7.1-11.9% of sodium carboxylmethyl cellulose), Avicel® RC-581 (containing 8.3-13.8% of sodium carboxymethyl cellulose), Avicel® RC-591 (containing 8.3-13.8% of sodium carboxymethyl cellulose) and Avicel® CL-611 (containing 11.3-18.8% of sodium carboxymethyl cellulose). All types are hygroscopic powders, which are insoluble in organic solvents and dilute acids, and partially soluble in both dilute alkali and water (due to the sodium carboxymethyl cellulose component). In view of the above-mentioned properties, it is surprising that all four types may be used to prepare the granulate according to the invention, which allows for the preparation of fast-disintegrating compositions, having a fast release of the active ingredient. Of the above-mentioned grades preferably the Avicel® RC 581 type is used and most advantageously the Avicel® RC 591 type is incorporated in the granulate. The water dispersible celluloses can be used in an amount up to ≤ 15 wt% based on the weight of the active ingredient, but preferably the said celluiose is used in a concentration of between 1 and 7.5 wt% and more advantageously in a concentration ranging from 2 to 5 wt%, all percentages based on the weight of the active ingredient. Surprisingly, in the present inventors' experience the preparation of a granulate out of an active ingredient and corresponding amounts of microcrystalline cellulose and sodium carboxymethyl cellulose respectively, did not result in fast-disintegrating and fast-dissolving compositions.

The active ingredients, to be incorporated in the granulate, have a solubility in water of 1:>10 at room temperature, but preferably the solubility is 1:≥100. Advantageously the active ingredient is a drug which has to be administered in a high doses to be effective. Examples of such drugs are antimicrobial compounds in general, selected from the group of beta-lactam compounds, such as the penicillins (amoxicillin, ampicillin) and cephalbsporins (cefaclor); the group of macrolides, such as erymromycin and josamycin; the group of sulphonamides, such as sulphamethoxazole; the group of hydroxyquinolones and quinolones, such as ciprofloxacin; the group of imidazoles and nitroimidazoles, such as metronidazole and tinidazole; and various other compounds, such as nalidixic acid and nitrofurantoin. However other drugs, not belonging to the antimicrobial compounds, can also be succesfully granulated according to the present invention: antacids, such as hydrotalcite, analgesic and anti-inflammatory drugs, such as ibuprofen, acetaminophen and acetylsalicylic acid, antidiabetic agents, such as tolbutamide, antimalarials, such as amodiaquine hydrochloride, tuberculostatics and tuberculocides, such as rifampicin, anticonvulsants, such as carbamazepine, and dopaminergic agents, such as levodopa. Alternatively the active ingredient is a non-drug, but a compound which is able to enhance the efficacy of a drug if co-administered (either simultaneously or consecutively) with it. An example thereof is a beta-lactamase inhibitor, such as clavulanic acid or a pharmaceutically acceptable salt thereof, which showed a significantly improvement of the efficacy of a beta-lactam antibiotic compound. The high active ingredient content of the granulate also allows for combining active ingredients, such as drugs, in the same granulate, if no objections as to incompatibilities arise.

The granulates according to the present invention are prepared according to conventional wet granulation methods, e.g. as taught by I.M. Jalal et al. Preferably the active ingredient and water dispersible cellulose are blehded in a suitable mixer, which may be a planetary mixer, a high shear or a high speed mixer. Mixing times depend on the specific apparatus used. Thereafter water or an aqueous solution is added until the material is sufficiently moistened. This may be done in the mixer used for blending the components or alternatively the powder blend may be transferred to a fluidised bed granulator, wherein the aqueous solution is sprayed onto the materials. The amount of water used may range from 20 to 30 wt%, based on the weight of the granulate, After partial drying the wet mass is passed through a first screen and subsequently further dried in a fluidized bed dryer at an air inlet temperature of between 40 and 60°C. After drying the granules until the required free moisture content, these are passed through a second screen. Alternatively the wet mass is transferred to a fluidized bed dryer without wet screening. After drying the granules are passed through a first and a second screen and optionally a third screen respectively.

Whereas many of the above-mentioned active ingredients have unsatisfactory flow-properties, the granulates according to the invention have sufficient to good flow properties. Due to a good bulk volume and a proper Hausner ratio they can be easily processed into compositions. Disintegration and dissolution behaviour of the granulates are also satisfactory.

The granulates according to the invention are very versatile in their applications. Optionally after addition of suitable excipients, such as lubricants, flow-promotors, anti-adherents, etc., the granulates can be filled into capsules or sachets. The compositions, based on the above granulate, preferably contain the granulate in an amount of ≥ 80 wt% in order to obtain high-dosed compositions. However, due to the high active ingredient content and limited number of excipients required for obtaining fast-disintegrating and fast-dissolving compositions, blending of a granulate containing a particular active ingredient with another active ingredient external to the granulate can be very favourable in case a combination of drugs or a combination of a drug and a compound enhancing the drug's activity is to be considered. It goes without saying that the granulate can also be incorporated in a dosage-form together with a greater part of excipients. This might be very favourable in case taste-masking of an active ingredient is required.

In order to prepare fast-disintegrating and fast-dissolving compositions, containing a high amount of drug, the granulate is advantageously blended with a first disintegrant and a second disintegrant. The first disintegrant is preferably a cellulose product, which is microcrystalline cellulose (Avicel® PH- 101, Avicel® PH 102), microfine cellulose or a mixture thereof. The second disintegrant may be starch, such as Star-X 1500®, or a starch derivative, such as sodium starch glycolate, but is preferably selected from the group of superdisintegrants, such as cross-linked polyvinylpyrrolidone and low-substituted hydroxypropyl cellulose. Both the first and the second disintegrant are advantageously added in an amount ranging from about 2, e.g. 1.5 wt%, to 8 wt%, more preferably 3-6 wt%, the percentage based on the weight of the composition. The ratio of the amount of the first and the second disintegrant in the composition may range from <4:1 to 1:4, but preferably is 1:1. The compositions may additionally contain flavours, sweetening agents, such as saccharinic acid, the sodium salt thereof or aspartame, lubricants, such as colloidal silicon dioxide, stearic acid or a salt thereof, but preferably such excipients are added in an amount of less than 4 wt%, the percentage based on the weight of the composition.

Alternatively, fast-disintegrating and fast-dissolving compositions can be prepared out of two granulates, containing a different active ingredient. It can also be advantageous to prepare a granulate out of some of the excipients to be added to the granulate according to the invention. E.g. by means of dry granulating methods a granulate can be advantageously prepared out of the flavours, sweetening agents and one or both of the disintegrants. Optionally the granulate can be blended with a drug or a compound which enhances the activity of the active ingredient incorporated in the granulate, such as a beta-lactamase inhibitor in case the intra-granular active ingredient is a beta-lactam compound.

By fast disintegration is meant a disintegration time in water of room temperature (20°C) of less than 2 minutes and preferably less than one minute. For the assessment thereof the method according to the European Pharmacopoeia is used however with a further modification of the movement (22 mm instead of 55 mm), simulating a users' situation. Fast dissolution is to be considered as >95% of the drug dissolved in a suitable dissolution medium, e.g. as prescribed in the U.S. Pharmacopoeia 1995 for each particular drug, of 37°C after 30 minutes. Preferably 90% of the drug has been dissolved after 10 minutes (same conditions).

The compositions according to the present invention show many advantages. In a bioequivalence study an amoxicillin- and a cefaclor-containing tablet according to the present invention, either taken as such or after prior dispersal in water, have proved to be equivalent to commercially available compositions. Furthermore, the considerable variation in disintegration time and dissolution rate between compositions, containing a different medicament in a similar carrier, as shown by the prior art compositions described by Jalal et al., has been reduced. By using the high-dosed granulate a considerable reduction of the volume of the dosage-form has been achieved. The reduced size is desirable both from a manufacturing and handling standpoint and a patient acceptability standpoint. The advantages thereof are manifold: a patient can more easily swallow a tablet composition, as a consequence of which patient compliance will be increased. Due to the reduction of the amount of excipients to be used, the new compositions also have advantages both from an economical point of view, such as considerable savings on production costs and a reduction of packaging materials, and from an environmental point of view, such as a reduction of waste from e.g. aluminum and polyvinyl-chloride, which packaging materials cannot be recycled.

The following examples further illustrate the invention.

### EXAMPLES

### Example 1

970 g of cefaclor (as monohydrate) and 30 g of dispersible cellulose (Avicel®RC591) were mixed for 5 minutes in a planetary mixer. Gradually about 320 ml of water was added to this blend and mixing was continued for another 5 minutes. The wet granulate was dried in a fluidised bed dryer at an air inlet temperature of 50 °C and subsequently sieved through a 1.00 mm and 0.630 mm screen respectively.

### Example 2

864 g of the granulate obtained according to example 1 was mixed with 98 g of a mixture of microcrystalline cellulose and cross-linked polyvinylpyrrolidone (1:1), flavours and sweetening agents in a TURBULA-mixer for 10 minutes. After a lubricant was added mixing was continued for another 3 minutes and the mixture was compressed voter tablets, haying a mean weight of 625 mg.

The tablets obtained had the following characteristics:

| | |
|---|---|
| Friability | <0.01% |
| Hardness | 6.9 kP |
| Mean tablet weight | 627 mg |
| Disintegration time (in water of 20°C) | 22 seconds |
| Dissolution | 98% cefaclor dissolved after 10 minutes |
| | 99% cefaclor dissolved after 30 minutes |

### Example 3

| | |
|---|---|
| Cefaclor monohydrate | 524.0 mg |
| dispersible cellulose (Avicel®RC591) | 15.7 mg |
| microcrystalline cellulose | 13.5 mg |
| low-substituted hydroxypropyl cellulose | 13.5 mg |
| flavour | 9.1 mg |
| sweetening agent | 9.1 mg |
| lubricants | 9.2 mg |

A granulate and tablets were prepared out of the above components according to the methods described in examples 1 and 2. The tablets so obtained had the following characteristics:

| | |
|---|---|
| Friability | <0.01% |
| Hardness | 7.4 kP |
| Mean tablet weight | 597 mg |
| Disintegration timie (in water of 20°C) | 100 seconds |
| Dissolution | 92 % cefaclor dissolved after 10 minutes |
| | 96 % cefaclor dissolved after 30 minutes |

### Example 4

| | |
|---|---|
| Amoxicillin (as trihydtate) | 86.9 % |
| dispersible cellulose (Avicel®RC591) | 2.6 % |
| cross-linked polyvinylpyrrolidone | 3.8 % |
| microcrystalline cellulose | 3.8 % |
| flavours | 1.6 % |
| sweetening agent | 1.0 % |
| lubricant | 0.4 % |

A granulate and tablets were prepared out of the above components according to the methods described in examples 1 and 2. The tablets obtained in this way had the following characteristics:

| | |
|---|---|
| disintegration time (in water of 20° C) | 48-50 seconds |
| Dissolution | 98.5% of amoxicillin dissolved after 30 minutes |
| Mean tablet weight | 1330 mg |
| Hardness | 20kP |
| Friability | <0.01% |

### Example 5

A granulate and tablets were prepared according to the methods described in examples 1 and 2 out of the same components as mentioned in example 4, but amoxicillin trihydrate was also substitued by another drug selected from ampicillin anhydrate, acetaminophen, sulphamethoxazole, ibuprofen and ciprofloxacine. The tablets obtained in this way had the following characteristics:

| drug | hardness(kP) | disintegration time in water of 20°C(s) | dissolution (% after 10 minutes) | disssolution (% after 30 minutes) |
|---|---|---|---|---|
| amoxicillin trihydrate | 7.5 | 45 | 92.0 | 97.2 |
| ampicillin anhydrate | 7.1 | 41 | 86.7 | 97.2 |
| acetaminophen | 3.7 | 25 | 93.7 | 102.0 |
| sulphamethoxazole | 7.3 | 36 | 66.9 | 86.2 (after 20 minutes) |
| ibuprofen | 5.5 | 54 | 98.0 | 101.8 |
| ciprofloxacine | 6.0 | 32 | 95.9 | 99.3 |
| Note: the dissolution of all tablets, except for the amoxicillin-containing ones, was assessed according to the methods described in the USP '95. | | | | |

### Example 6

Like in example 5 a granulate and tablets were prepared according to the methods described in examples 1 and 2 out of the same components as mentioned in example 4, but amoxicillin trihydrate was also substituted by another drug selected from ampicillin anhydrate, sulphamethoxazole, erythromycine ethyl succinate and ciprofloxacine. The tablets obtained in this way had the following characteristics:

| drug | hardness (kP) | disintegration time in water of 20°C(s) | hardness (kP) | disintegration time in water of 20°C(s) |
|---|---|---|---|---|
| amoxicillin trihydrate | 10.4 | 47 | 14.7 | 47 |
| ampicillin anhydrate | 10.6 | 40 | 15.4 | 59 |
| sulphamethoxazole | 10.1 | 57 | 13.8 | 66 |
| erythromycine ethyl succinate | 10.6 | 67 | 13.9 | 111 |
| ciprofloxacine | 9.1 | 34 | 14.1 | 65 |

### Example 7

A granulate and tablets were prepared according to the methods described in examples 1 and 2 out of the components as mentioned in example 4, but another type of dispersible cellulose was used. The tablets obtained in this way had the following characteristics:

| type of dispersible cellulose | hardness (kP) | disintegration time in water of 20°C (s) |
|---|---|---|
| Avicel® RC-501 | 6.8 | 77 |
| Avicel® CL-611 | 6.4 | 93 |

### Example 8

| | |
|---|---|
| granulate (= amoxicillin trihydrate/dispersible cellulose (Avicel®RC581) 98/2) | 89.6 % |
| cross-linked polyvinyl pyrrolidone | 3.9 % |
| microcrystalline cellulose | 3.9 % |
| flavours | 1.6 % |
| sweetening agent | 1.0 % |

A granulate and tablets were prepared according to the methods described in examples 1 and 2. The tablets so obtained had a disintegration time in water of 20°C which was 92 seconds. The observed hardness of the tablet was 7 kP.

| | |
|---|---|
| granulate (= amoxicillin trihydrate/dispersible cellulose (Avicel®RC581)97/3) | 84.75 % |
| cross-linked polyvinyl pyrrolidone | 4 % |
| microcrystalline cellulose | 11 % |
| lubricant | 0.25 % |

A granulate and tablets were prepared according to the methods described in examples 1 and 2. The tablets so obtained had the following characteristics:

| hardness (kP) | disintegration time in water of 20°C (s) |
|---|---|
| 6 | 102 |
| 12 | 120 |
| 18 | 114 |

### Example 10

| | |
|---|---|
| granulate (= amoxicillin trihydrate/ dispersible cellulose (Avicel®RC581) 96/4) | 84 % |
| cross-linked polyvinyl pyrrolidone | 4 % |
| microcrystalline cellulose | 8.7 % |
| lubricant | 3.3 % |

A granulate and tablets were prepared according to the methods described in examples 1 and 2. The tablets so obtained had the following characteristics:

| hardness (kP) | disintegration time in water of 20°C(s) |
|---|---|
| 5 | 94 |
| 9 | 90 |
| 15 | 115 |

### Example 11

| | |
|---|---|
| granulate (= amoxicillin trihydrate/ dispersible cellulose (Avicel®RC581) 92/8) | 89.3 % |
| cross-linked polyvinyl pyrrolidone | 4.0% |
| microcrystalline cellulose | 6.4% |
| lubricant | 0.2 % |

A granulate and tablets were prepared according to the methods described in examples 1 and 2. The tablets so obtained had a disintegration time in water of 20°C which was 80 seconds. The observed hardness of the tablet was 6 kP.

### Example 12

A granulate and tablets were prepared according to the methods described in examples 1 and 2 out of the components as mentioned in example 4, but the ratio of amoxicillin trihydrate and dispersible cellulose (Avicel® RC-591) in the granulate was varied. The tablets so obtained had the following characteristics:

| ratio amoxicillin trihydrate/ dispersible cellulose (Avicel® RC-591) | hardness (kP) | disintegration time in water of 20°C (s) |
|---|---|---|
| 95/5 | 7.3 | 77 |
| 92.5/7.5 | 7.1 | 87 |
| 85/15 | 6.5 | 127 |

### Example 13

| | |
|---|---|
| granulate (= amoxicillin trihydrate/ dispersible cellulose (Avicel®RC591) 95/5) | 84.1 % |
| cross-linked polyvinyl pyrrolidone | 6.3 % |
| microcrystalline cellulose | 6.3 % |
| flavours | 1.6 % |
| sweetening agent | 1.0 % |
| lubricant | 0.7 % |

A granulate and tablets were prepared according to the methods described in examples 1 and 2 out of the above components. The tablets so obtained had the following characteristics:

| hardness (kP) | disintegration time in water of 20°C (s) |
|---|---|
| 7 | 52-56 |
| 11 | 56-61 |
| 18 | 58-62 |

### Example 14

A first granulate was made out of 300 g of amoxicillin trihydrate and dispersible cellulose (Avicel® RC-591) according to the method described in example 1. A second granulate was made by blending microcrystalline cellulose (59.5 wt%), flavours (23.3 wt%) and a sweetening agent (5.5 wt%), compressing tablets having a hardness of 1.5-2 kP out of the blend and breaking up the same tablets to form a granulate by means of a Frewitt oscillating granulator. The first and second granulate were blended and subsequently mixed with cross-linked polyvinylpyrrolidone and a lubricant, whereafter tablets were compressed out of the mixture so obtained. The tablets had the following characteristics:

| Composition of tablet | % | % | % | % |
|---|---|---|---|---|
| granulate 1 | 79.6 | 84.6 | 89.5 | 94.5 |
| granulate 2 | 12.5 | 9.4 | 6.4 | 3.1 |
| cross-linked polyvinylpyrrolidone | 7.4 | 5.6 | 3.8 | 1.9 |
| lubricant | 0.4 | 0.4 | 0.4 | 0.5 |
| properties of tablet | | | | |
| baldness (kP) | 5.0 | 7.3 | 7.5 | 6.8 |
| disintegration time in water of 20°C | 31.8 | 43.8 | 45.0 | 92.3 |

### Example 15

A granulate and tablets were prepared according to the methods described in examples 1 and 2 out of the components as mentioned in example 4, but the second extra-granular disintegrant cross-linked polyvmylpyrrolidone was substituted by another disintegrant, selected from starch (Star-X 1500®), sodium starch glycolate, low-substituted hydroxypropyl cellulose and maize starch. The tablets so obtained had the following characteristics:

| second extragranular disintegrant | hardness (kP) | disintegration time in water of 20°C (s) |
|---|---|---|
| starch (Star-X 1500®) | 5.8 | 73 |
| sodium starch glycolate | 4.8 | 124 |
| low-substituted hydroxypropyl cellulose | 5.1 | 53 |
| maize starch | 5.9 | 71 |

## Claims

1. Granulate for the preparation of fast-disintegrating and fast-dissolving compositions, containing an active ingredient, having a solubility in water of 1:>10, in admixture with a water dispersible cellulose, which is a microcrystalline cellulose and sodium carboxymethyl cellulose, **characterised in that** the water dispersible cellulose is present in an amount of ≤ 15 wt%, the percentage based on the weight of the active ingredient.

2. Granulate according to claim 1, **characterised in that** it contains 1-7.5 wt% of the water dispersible cellulose.

3. Granulate according to claim 1 or 2, **characterised in that** it contains 2-5 wt% of the water dispersible cellulose.

4. Granulate according to any one of claims 1-3, **characterised in that** the water dispersible cellulose contains 8.3-13.8% of sodium carboxymethyl cellulose.

5. Granulate according to any one of claims 1-4, **characterised in that** it has been prepared by wet granulation and subsequent sieving.

6. Fast-disintegrating and fast-dissolving composition, containing at least one granulate according to any one of claims 1-5 in admixture with a first disintegrant and a second disintegrant and further pharmaceutically acceptable excipients, and optionally a drug or a compound, enhancing the activity of the active ingredient incorporated in the granulate.

7. Composition according to claim 6, **characterised in that** one or more of the granulates according to any one of claims 1-5 are present in a total amount of ≥ 80 wt%, in admixture with 2-8 wt% of a first disintegrant and 2-8 wt% of a second disintegrant, the percentages based on the weight of the composition.

8. Composition according to claim 7, **characterised in that** the first disintegrant is microcrystalline cellulose, microfine cellulose or a mixture thereof and the second disintegrant is starch or a starch derivative, cross-linked polyvinylpyrrolidone or low-substituted hydroxypropyl cellulose.

9. Composition according to claim 7 or 8, **characterised in that** the first disintegrant and the second disintegrant are present in a ratio of 1:1.

## Patentansprüche

1. Granulat zur Herstellung von schnell zerfallenden und sich schnell auflösenden Zusammensetzungen, die einen Wirkstoff, der in Wasser eine Löslichkeit von 1:>10 aufweist, zusammen mit einer in Wasser dispergierbaren Zellulose, die eine mikrokristalline Zellulose und Natriumcarboxymethyl-Zellulose ist, enthalten, **dadurch gekennzeichnet, dass** die in Wasser dispergierbare Zellulose in einer Menge ≤ 15 Gew.-% vorliegt, wobei die Prozentzahlen auf das Gewicht des Wirkstoffs bezogen sind.

2. Granulat nach Anspruch 1, **dadurch gekennzeichnet, dass** es 1-7,5 Gew.-% der in Wasser dispergierbaren Zellulose enthält.

3. Granulat nach Anspruch 1 oder 2, **dadurch gekennzeichnet, dass** es 2-5 Gew.-% der in Wasser dispergierbaren Zellulose enthält.

4. Granulat nach Anspruch 1-3, **dadurch gekennzeichnet, dass** die in Wasser dispergierbare Zellulose 8,3-13,8 % Natriumcarboxymethyl-Zellulose enthält.

5. Granulat nach Anspruch 1-4, **dadurch gekennzeichnet, dass** es durch Nassgranulierung und anschließendes Sieben hergestellt worden ist.

6. Schnell zerfallende und sich schnell auflösende Zusammensetzung enthaltend mindestens ein Granulat nach einem der Ansprüche 1-5 zusammen mit einem ersten Sprengmittel und einem zweiten Sprengmittel und weiteren pharmazeutisch annehmbaren Trägern und gegebenenfalls ein Arzneimittel oder eine Verbindung, das bzw. die die Aktivität des in das Granulat eingearbeiteten Wirkstoffs erhöht.

7. Zusammensetzung nach Anspruch 6, **dadurch gekennzeichnet, dass** das bzw. die Granulat(e) nach einem der Ansprüche 1-5 in einer Menge von ≥ 80 Gew.-% zusammen mit 2-8 Gew.-% des ersten Sprengmittels und 2-8 Gew.-% des zweiten Sprengmittels vorliegen, wobei die Prozentzahlen auf das Gewicht der Zusammensetzung bezogen sind.

8. Zusammensetzung nach Anspruch 7, **dadurch gekennzeichnet, dass** das erste Sprengmittel mikrokristalline Zellulose, mikrofeine Zellulose oder ein Gemisch daraus ist und das zweite Sprengmittel Stärke oder ein Stärkederivat, quervernetztes Polyvinylpyrrolidon oder niedrig-substituierte Hydroxypropyl-Zellulose ist.

9. Zusammensetzung nach Anspruch 7 oder 8, **dadurch gekennzeichnet, dass** das erste Sprengmittel und das zweite Sprengmittel in einem Verhältnis von 1:1 vorliegen.

## Revendications

1. Granulé destiné à la préparation de compositions à désintégration rapide et à dissolution rapide, contenant un ingrédient actif, ayant une solubilité dans l'eau de 1:>10, en mélange avec une cellulose dispersible dans l'eau qui est une cellulose microcristalline et la carboxyméthylcellulose sodique, **caractérisé en ce que** la cellulose dispersible dans l'eau est présente en une quantité de ≤ 15% en poids, le pourcentage étant basé sur le poids de l'ingrédient actif.

2. Granulé suivant la revendication 1, **caractérisé en ce qu'**il contient 1 à 7,5% en poids de cellulose dispersible dans l'eau.

3. Granulé suivant la revendication 1 ou 2, **caractérisé en ce qu'**il contient 2 à 5% en poids de cellulose dispersible dans l'eau.

4. Granulé suivant l'une quelconque des revendications 1 à 3, **caractérisé en ce que** la cellulose dispersible dans l'eau contient 8,3-13,8% de carboxyméthylcellulose sodique.

5. Granulé suivant l'une quelconque des revendications 1 à 4, **caractérisé en ce qu'**il a été préparé par granulation par voie humide et tamisage ultérieur.

6. Composition à désintégration rapide et à dissolution rapide, contenant au moins un granulé suivant l'une quelconque des revendications 1 à 5, en mélange avec un premier désintégrant et un deuxième désintégrant et des excipients pharmaceutiquement acceptables et, facultativement, un médicament ou un composé, augmentant l'activité de l'ingrédient actif incorporé au granulé.

7. Composition suivant la revendication 6, **caractérisée en ce qu'**un ou plusieurs des granulés suivant l'une quelconque des revendications 1 à 5 sont présents en une quantité totale de ≥ 80% en poids, en mélange avec 2 à 8% en poids d'un premier désintégrant et 2 à 8% en poids d'un deuxième désintégrant, les pourcentages étant basés sur le poids de la composition.

8. Composition suivant la revendication 7, **caractérisée en ce que** le premier désintégrant est la cellulose microcristalline, la cellulose microfine ou un mélange de celles-ci et **en ce que** le deuxième désintégrant est l'amidon ou un dérivé de l'amidon, la polyvinylpyrrolidone réticulée ou l'hydroxypropyl-cellulose faiblement substituée.

9. Composition suivant la revendication 7 ou 8, **caractérisée en ce que** le premier désintégrant et le deuxième désintégrant sont présents en un rapport de 1:1.
